# FASCICULE DE BREVET EUROPEEN

(11) **EP 1 248 843 B1**
(45) Date de publication et mention de la délivrance du brevet: **28.04.2010**
(21) Numéro de dépôt: 01907652.0
(22) Date de dépôt: 19.01.2001
(51) Int. Cl.: C12N 15/12, C12N 5/10, C07K 14/705, C07K 16/28, C12Q 1/68, G01N 33/566, A61K 31/70, A61K 38/17

(54) **GENE NPHS2 IMPLIQUE DANS LE SYNDROME NEPHROTIQUE CORTICO-RESISTANT**
DAS NPHS2 GEN, WELCHES IM CORTICORESISTENTEN NEPHROTISCHEN SYNDROME EINE ROLLE SPIELT
NPHS2 GENE INVOLVED IN THE CORTICO-RESISTANT NEPRHOTIC SYNDROME, PROTEIN ENCODED BY SAID GENE AND DIAGNOSTIC AND THERAPEUTIC USES

(30) Priorité: 20.01.2000 FR 0000709
(43) Date de publication de la demande: 16.10.2002
(73) Titulaire: INSTITUT NATIONAL DE LA SANTE ET DE LA RECHERCHE MEDICALE (INSERM), 75013 Paris (FR)
(72) Inventeur: ANTIGNAC, Corinne, F-75018 Paris (FR); BOUTE, Nicolas, F-78120 Rambouillet (FR)
(74) Mandataire: Bernasconi, Jean Raymond
(86) Numéro de dépôt international: PCT/FR2001/000188
(87) Numéro de publication internationale: WO 2001/053347

(56) Documents cités:
- DATABASE SÉQUENCES EMBL [en ligne] Code d'accès AI913530, 30 juillet 1999 (1999-07-30) STRAUSBERG R.: "EST; H. sapiens kidney cDNA clone IMAGE:2297835" XP002149359
- BOUTE N. ET AL.: "MPHS2, encoding the glomerular protein podocin, is mutated in autosomal recessive steroid-resistant nephrotic syndrome." NATURE GENETICS, vol. 24, no. 4, avril 2000 (2000-04), pages 349-354, XP000946884
- DATABASE EMBL SEQUENCES [en ligne] Accession No. AL160286, 13 mars 2000 (2000-03-13) "Human DNA sequence from clone RP11-545A16" XP002168082
- FUCHSHUBER A. ET AL.: "Mapping a gene (SRN1) to chromosome 1q25-q31 in idiopathic nephrotic syndrome confirms a distinct entity of autosomal recessive nephrosis." HUM. MOL. GENET., vol. 4, no. 11, novembre 1995 (1995-11), pages 2155-2158, XP000946841

## Description

On décrit ici un nouveau gène, appelé NPHS2, qui code pour une protéine impliquée dans le syndrome néphrotique cortico-résistant.

Le syndrome néphrotique idiopathique est une pathologie qui apparaît principalement chez l'enfant, et qui se caractérise par une protéinurie massive, et des changement histologiques non spécifiques du rein incluant parfois une sclérose glomérulaire segmentaire et focale (FSGS). Ces caractéristiques sont associées avec un effacement diffus des pédicelles des podocytes observé en microscopie électronique (Broyer *et al*., 1998), révélateur des syndromes néphrotiques quelle que soit leur cause. La plupart des cas répondent à une thérapie à base de stéroïdes et ont un bon pronostic, mais environ 20% sont résistants aux stéroïdes et évoluent vers l'insuffisance rénale terminale, conduisant à une sclérose glomérulaire complète. On parle alors de syndrome néphrotique cortico-résistant.

L'ultrafiltration des macromolécules du plasma durant la formation de l'urine primaire dans le glomérule est l'une des fonctions centrales du rein humain. La paroi capillaire structurellement complexe qui est responsable de cette fonction est composée d'une membrane basale recouverte d'un endothélium fenestré sur sa surface interne et par des cellules épithéliales spécialisées (podocytes) qui forment des pédicelles sur la surface externe. On observe dans un grand nombre de maladies acquises ou héréditaires un dysfonctionnement du filtre glomérulaire résultant en une perte excessive des protéines plasmatiques, menant à un syndrome néphrotique, puis éventuellement à une insuffisance rénale terminale.

L'étude des maladies génétiques affectant la barrière de filtration fournit des modèles utiles pour comprendre la physiopathologie du processus de filtration glomérulaire. Plusieurs de ces affections héréditaires avec protéinurie et syndrome néphrotique ont été décrites. Le plus sévère est le syndrome néphrotique congénital de type finlandais (CNF), qui est une maladie récessive autosomique avec une forte protéinurie *in utero,* un syndrome néphrotique à la naissance conduisant habituellement à une insuffisance rénale terminale durant les deux premières années de la vie. Le CNF est provoqué par des mutations dans le gène NPHS1 (Kestilä, 1998). Par ailleurs, des cas de protéinurie familiale ou de syndrome néphrotique avec des lésions histologiques de sclérose glomérulaire segmentaire ou focale (FSGS) ont été décrits chez des patients plus âgés, en particulier à l'âge adulte. Deux locus génétiques pour la FSGS dominante autosomique ont été cartographiés respectivement sur le locus 19q13 proche du locus du gène NPHS1 (Mathis *et al*., 1998) et sur le locus 11q21-q22 (Winn *et al*., 1999).

En 1995, une nouvelle entité de syndrome néphrotique corticorésistant de transmission autosomique récessive a été caractérisée selon les critères suivants : début précoce entre trois mois et cinq ans, résistance à une thérapie à base de stéroïdes, évolution vers l'insuffisance rénale terminale avant l'âge de dix ans, absence de récidive après transplantation rénale et absence de quelconque désordre extra-rénal. Histologiquement, on n'observe que des modifications minimes dans les biopsies précoces mais une FSGS est généralement présente à des stades ultérieurs. Un locus génétique impliqué dans ce syndrome néphrotique corticorésistant, a été cartographié dans la région 1q25-q31 entre les marqueurs D1S452 et D1S466, cette région s'étendant sur environ 12 cM (Fuchshuber, 1995). Cette localisation a été confirmée par une autre équipe (Lench *et al*., 1998) et plus récemment, une liaison à cette région a également été démontrée dans une famille présentant une FSGS débutant à l'âge adulte (Tsukaguchi *et al*., 1999).

Les auteurs de la présente invention sont maintenant parvenus à identifier de manière précise un nouveau gène impliqué dans l'entité de syndrome néphrotique cortico-résistant décrite ci-dessus. Ce gène a d'abord été appelé SRN1 puis a été renommé NPHS2.

En annexe se trouve une liste de séquences, dans laquelle la séquence SEQ ID n° 1 représente le fragment d'ADNc du gène NPHS2 chez l'homme correspondant au cadre de lecture ouvert (ORF). Cet ORF contient 1149 bases et code pour une protéine de 383 acides aminés, dénommée podocine, dont la séquence est présentée SEQ ID n° 2.

Les séquences SEQ ID n° 3 à SEQ ID n° 10 représentent des fragments de l'ADN génomique du gène NPHS2 humain incluant respectivement 8 exons (en caractères gras dans le listing annexé), comme suit :
SEQ ID n° 3 :
   Il y a 683 paires de bases avant l'ATG. Les clones d'ADNc obtenus par criblage d'une banque d'ADNc de rein foetal humain (banque Clontech clonée dans le phage λgt11) commencent généralement entre les bases 615 et 619. Il y a 274 paires de bases de l'ATG au site d'épissage (exon 1), puis 147 paires de bases de séquences introniques.
SEQ ID n° 4 :
   Il y a 151 paires de bases d'intron, puis 104 paires de bases de codant (exon 2), puis 123 paires de bases d'intron.
SEQ ID n° 5:
   Il y a 336 paires de bases d'intron, puis 73 paires de bases de codant (exon 3), puis 291 paires de bases d'intron.
SEQ ID n° 6 :
   Il y a 187 paires de bases d'intron, puis 83 paires de bases de codant (exon 4), puis 90 pb d'intron.
SEQ ID n° 7 :
   Il y a 250 paires de bases d'intron, puis 204 paires de bases de codant (exon 5), puis 195 paires de bases d'intron.
SEQ ID n° 8 :
   Il y a 367 paires de bases d'intron, puis 56 paires de bases de codant (exon 6), puis 169 paires de bases d'intron.
SEQ ID n° 9 :
   Il y a 327 pb d'intron, puis 79 paires de bases de codant (exon 7), puis 310 paires de bases d'intron.
SEQ ID n° 10 :
   Il y a 285 paires de bases d'intron, puis 911 paires de bases de séquence d'ADNc jusqu'au site de polyadénylation utilisé (exon 8). Le codon stop est en position 562, puis 109 paires de bases de séquences génomiques supplémentaires couvrant les autres sites potentiels de polyadénylation.

La séquence SEQ ID n° 11 couvre une partie de l'exon 5, les exons 6 et 7 et une grande partie de l'exon 8 (de la base 9 792 de l'ADNc).

Les séquences SEQ ID n° 12 à n° 27 sont des amorces utiles pour l'amplification des séquences humaines.

La séquence SEQ ID n° 28 est la séquence d'ADNc de podocine de rat, la séquence SEQ ID n° 29 étant la séquence d'acides aminés correspondante. La présente invention concerne un acide nucléique tel que défini dans les revendications 1, 4, 5, 6 et 9.

Par ailleurs, on décrit, un acide nucléique isolé, dont la séquence est choisie parmi SEQ ID n° 3 à SEQ ID n° 10, ou une séquence homologue, définie comme
i) une séquence identique à au moins 70% de la séquence SEQ ID n° 3 à SEQ ID n°10 ; ou
ii) une séquence hybridant avec la séquence SEQ ID n° 3 à SEQ ID n° 10 ou leurs séquences complémentaires, dans des conditions stringentes d'hybridation.

La présente invention a également pour objet un acide nucléique isolé, comprenant la séquence SEQ ID n° 1 ou 28, ou une séquence homologue, définie comme
i) une séquence identique à au moins 70% de la séquence SEQ ID n° 1 ; ou
ii) une séquence hybridant avec la séquence SEQ ID n° 1 ou sa séquence complémentaire, dans des conditions stringentes d'hybridation, ou
iii) une séquence codant pour le polypeptide, dénommé podocine, tel que défini précédemment.

De préférence, une séquence nucléotidique homologue selon l'invention est identique à au moins 75 % des séquences SEQ ID n°1, de préférence encore au moins 85 %, ou au moins 90 %.

De manière préférentielle, une séquence nucléotidique homologue hybride spécifiquement aux séquences complémentaires des séquences SEQ ID n° 1, 3 à 10, et 28 dans des conditions stringentes. Les paramètres définissant les conditions de stringence dépendent de la température à laquelle 50% des brins appariés se séparent (Tm).

Pour les séquences comprenant plus de 30 bases, Tm est définie par la relation : Tm=81,5+0,41(%G+C)+16,6Log(concentration en cations) - 0,63(%formamide) -(600/nombre de bases) (Sambrook *et al*., 1989).

Pour les séquences de longueur inférieure à 30 bases, Tm est définie par la relation : Tm= 4(G+C) + 2 (A+T).

Dans des conditions de stringence appropriées, auxquelles les séquences aspécifiques n'hybrident pas, la température d'hybridation est approximativement de 5 à 30°C, de préférence de 5 à 10°C en dessous de Tm, et les tampons d'hybridation utilisés sont de préférence des solutions de force ionique élevée telle qu'une solution 6xSSC par exemple.

Une séquence nucléotidique homologue à l'ORF représentée à la SEQ ID n° 1 ou 28 inclut toute séquence nucléotidique qui diffère de la séquence SEQ ID n° 1 ou 28 par mutation, insertion, délétion ou substitution d'une ou plusieurs bases, ou par la dégénérescence du code génétique, pour autant qu'elle code pour un polypeptide présentant l'activité biologique de la podocine, comme définie plus bas.

Parmi de telles séquences homologues, sont comprises les séquences des gènes de mammifères autres que l'homme, codant pour la podicine, de préférence d'un primate, d'un bovin, ovin ou porc, ou encore d'un rongeur, ainsi que les variants alléliques, ou séquences polymorphiques.

Le tableau ci-après présente un certain nombre de polymorphismes identifiés dans le gène NPHS2 :

### Polymorphismes identifiés dans le gène NPHS2

| **Exon** | **Polymorphisme** | **Position sur les séquences listées** |
|---|---|---|
| 1 | -51/ATGT>C | +19 sur SEQ ID n° 1 |
| | nt 102 (G>A) = G34G | +171 sur SEQ ID n° 1 |
| 2 | nt 288 (G>T) = S96S | +357 sut SEQ ID n° 1 |
| 5 | nt 686 (G>A) = R229Q | +755 sur SEQ ID n° 1 |
| 7 | 873 +7 A>G | +413 sur SEQ ID n° 9 |
| 8 | nt 954 (T>C) = A318A | +1023 sur SEQ ID n° 1 |
| | nt 1038 (A>G) = L346L | +1107 sur SEQ ID n° 1 |

La présente invention a également pour object un polypeptide isolé tel que défini dans la revendication 2 ou 3.

On décrit également ici un polypeptide isolé comprenant la séquence d'acides aminés SEQ ID n° 2 ou 29, ou une séquence homologue, définie comme
i) une séquence identique à au moins 70% de la séquence SEQ ID n° 2 ou 29 ; ou
ii) une séquence codée par une séquence d'acide nucléique homologue telle que définie dans la revendication 2 ii), c'est-à-dire une séquence d'acide nucléique hybridant avec la séquence SEQ ID n° 2 ou 29 ou sa séquence complémentaire, dans des conditions stringentes d'hybridation.

Plus généralement, par « séquence d'acides aminés homologue », on entend toute séquence d'acides aminés qui diffère de la séquence SEQ ID n°2 ou 29 par substitution, délétion et/ou insertion d'un acide aminé ou d'un nombre réduit d'acides aminés, notamment par substitution d'acides aminés naturels par des acides aminés non naturels ou pseudoacides aminés à des positions telles que ces modifications ne portent pas significativement atteinte à l'activité biologique de la podocine.

Lesdites substitutions sont de préférence des substitutions conservatives, c'est-à-dire des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires (tels que la glycine, l'alanine, la valine, la leucine, l'isoleucine, la proline, la phénylalanine, la méthionine, le tryptophane, et la cystéine).

De préférence, une telle séquence d'acides aminés homologue est identique à au moins 85 % de la séquence SEQ ID n° 2 ou 29, de préférence au moins 95 %.

L'homologie est généralement déterminée en utilisant un logiciel d'analyse de séquence (par exemple, Sequence Analysis Software Package of the Genetics Computer Group, University of Wisconsin Biotechnology Center, 1710 University Avenue, Madison, WI 53705). Des séquences d'acides aminés similaires sont alignées pour obtenir le maximum de degré d'homologie (i.e. identité). A cette fin, il peut être nécessaire d'introduire de manière artificielle des espaces (« gaps ») dans la séquence. Une fois l'alignement optimal réalisé, le degré d'homologie (i.e. identité) est établi par enregistrement de toutes les positions pour lesquelles les acides aminés des deux.séquences comparées sont identiques, par rapport au nombre total de positions.

"L'activité biologique de la podocine" se réfère au maintien de l'intégrité du filtre glomérulaire. L'absence ou l'altération de la podocine provoque la fuite de protéines au niveau du glomérule et par conséquent l'apparition d'une protéinurie.

Le polypeptide de la présente invention peut être synthétisé par toutes les méthodes bien connues de l'homme du métier. Le polypeptide de l'invention peut par exemple être synthétisé par les techniques de la chimie de synthèse, telles que la synthèse de type Merrifield qui est avantageuse pour des raisons de pureté, de spécificité antigénique, d'absence de produits secondaires non désirés et pour sa facilité de production.

Une podocine recombinante peut également être produite par un procédé, dans lequel un vecteur contenant un acide nucléique comprenant la séquence SEQ ID n° 1 ou n° 28 ou une séquence homologue est transféré dans une cellule hôte qui est mise en culture dans des conditions permettant l'expression du polypeptide correspondant.

La podocine produite peut ensuite être récupérée et purifiée.

Les procédés de purification utilisés sont connus de l'homme du métier. Le polypeptide recombinant obtenu peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono- ou polyclonaux spécifiques, etc.

La séquence d'acide nucléique d'intérêt, codant pour la podocine, peut être insérée dans un vecteur d'expression, dans lequel elle est liée de manière opérante à des éléments permettant la régulation de son expression, tels que notamment des promoteurs, activateurs et/ou terminateurs de transcription.

Les signaux contrôlant l'expression des séquences nucléotidiques (promoteurs, activateurs, séquences de terminaison...) sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium.

Les vecteurs de clonage et/ou d'expression selon la revendication 7 font également partie de la présente invention.

L'invention vise en outre les cellules hôtes transfectées, de manière transitoire ou stable, par ces vecteurs d'expression. Ces cellules peuvent être obtenues par l'introduction dans des cellules hôtes, procaryotes ou eucaryotes, d'une séquence nucléotidique insérée dans un vecteur tel que défini ci-dessus, puis la mise en culture desdites cellules dans des conditions permettant la réplication et/ou l'expression de la séquence nucléotidique transfectée.

Des exemples de cellules hôtes incluent notamment des cellules de mammifères, telles que les cellules COS-7, 293, MDCK, des cellules d'insectes telles que les cellules SF9, des bactéries telles que *E. coli* et des souches de levures telles que YRG2.

Les différentes séquence nucléotidiques décrites ici peuvent être d'origine artificielle ou non. Il peut s'agir de séquences d'ADN ou d'ARN, obtenues par criblage de banques de séquences au moyen de sondes élaborées sur la base des séquences SEQ ID n° 1 ou 28 et 3 à 10. De telles banques peuvent être préparées par des techniques classiques de biologie moléculaire, connues de l'homme de l'art.

Les séquences nucléotidiques décrites ici peuvent également être préparées par synthèse chimique, ou encore par des méthodes mixtes incluant la modification chimique ou enzymatique de séquences obtenues par criblage des banques.

Ces séquences nucléotidiques permettent la réalisation de sondes ou amorces, hybridant spécifiquement avec une séquence SEQ ID n° 1 ou 28, ou 3 à 10, selon l'invention, ou son brin complémentaire. Les conditions d'hybridation appropriées correspondent aux conditions de température et de force ionique usuellement utilisées par l'homme du métier, de préférence dans des conditions stringentes telles que définies précédemment. Ces sondes peuvent être utilisées comme outil de diagnostic *in vitro* pour la détection, par des expériences d'hybridation, notamment d'hybridation *"in situ",* de transcrits spécifiques du polypeptide de l'invention dans des échantillons biologiques ou pour la mise en évidence de synthèses aberrantes ou d'anomalies génétiques résultant d'un polymorphisme, de mutations ou d'un mauvais épissage.

Les acides nucléiques de l'invention utiles comme sondes comportent au minimum 10 nucléotides, préférentiellement au moins 20 nucléotides, préférentiellement encore au moins 100 nucléotides. Les acides nucléiques utiles comme amorces comportent au minimum 10 nucléotides, de préférence au moins 14 nucléotides, et préférentiellement moins de 40 nucléotides.

Plus précisément, on décrit ici un acide nucléique ayant au moins 10 nucléotides, qui hybride spécifiquement avec l'une des séquences d'acide nucléique SEQ ID n° 1 ou 28, ou 3 à 10 ou son complémentaire, dans des conditions stringentes d'hybridation.

De manière avantageuse, on peut utiliser comme sonde l'acide nucléique constitué de la séquence SEQ ID n° 11, qui couvre une partie de l'exon 5, les exons 6 et 7 et une grande partie de l'exon 8 (de la base 728 à la base 1792 de l'ADNc).

On peut par ailleurs utiliser comme amorce, pour une amplification (par exemple par PCR), les acides nucléiques constitués des séquences SEQ ID n°12 à SEQ ID n°27.

Préférentiellement, les sondes ou amorces de l'invention sont marquées, préalablement à leur utilisation. Pour cela, plusieurs techniques sont à la portée de l'homme du métier comme par exemple le marquage fluorescent, radioactif, chimioluminescent ou enzymatique.

Les méthodes de diagnostic *in vitro* dans lesquelles ces oligonucléotides sont mis en oeuvre pour la détection de mutations ou de remaniements génomiques, au niveau du gène NPHS2, sont incluses dans la présente description.

L'homme du métier connaît bien les méthodes standard pour analyser l'ADN contenu dans un échantillon biologique et pour diagnostiquer un désordre génétique. De nombreuses stratégies d'analyse génotypique sont disponibles (Antonarakis *et al*., 1989 ; Cooper *et al*., 1991).

De préférence, on peut utiliser la méthode DGGE (Electrophorèse sur gel de gradient dénaturant), la méthode SSCP (Polymorphisme de conformation simple brin) ou la méthode DHPLC (Chromatographie liquide haute performance dénaturante ; Kuklin *et al*., 1997; Huber *et al.,* 1995) pour détecter une anomalie dans le gène NPHS2. De telles méthodes sont de préférence suivies par un séquençage direct. Le procédé de RT-PCR peut être avantageusement mis en oeuvre pour détecter des anomalies dans le transcript de NPHS2, car il permet de visualiser les conséquences d'une mutation d'épissage provoquant la perte d'un ou plusieurs exons au niveau du transcrit, ou un épissage aberrant dû à l'activation d'un site cryptique. Ce procédé est également de préférence suivi d'un séquençage direct. Les procédés plus récemment développés utilisant des puces à ADN peuvent également être mis en oeuvre pour détecter une anomalie dans le gène NPHS2 (Bellis *et al*., 1997).

Le clonage du gène NPHS2 ainsi que l'identification de diverses mutations responsables du syndrome néphrotique cortico-résistant permettent d'envisager des diagnostics directs. La spécificité et la fiabilité de telles méthodes de diagnostic sont particulièrement appréciables pour un diagnostic prénatal. Les séquences d'acides nucléiques décrites ici représentent donc un outil particulièrement intéressant pour le conseil génétique.

La présente invention a donc pour objet l'utilisation selon la revendication 13.

L'invention a, par suite, pour objet un procédé de diagnostic *in vitro* d'un syndrome néphrotique cortico-résistant lié à une mutation du gène NPHS2, comprenant les étapes consistant à :
a1) mettre en présence un échantillon biologique contenant de l'ADN avec des oligonucléotides spécifiques permettant l'amplification de tout ou partie du gène NPHS2, défini comme comprenant une séquence d'acide nucléique selon la revendication 1 ;
b1) amplifier ledit ADN ;
c1) détecter les produits d'amplifcation ;
d1) comparer les produits d'amplification obtenus à ceux obtenus avec un échantillon contrôle, et détecter de cette manière une éventuelle anomalie dans ledit gène NPHS2, indicatrice d'un syndrome néphrotique cortico-résistant lié à une mutation du gène NPHS2 ;
ou, selon une alternative,
a2) mettre en présence un échantillon biologique contenant de l'ARN avec des oligonucléotides spécifiques permettant l'amplification de tout ou partie du transcrit du gène NPHS2, défini comme comprenant une séquence d'acide nucléique de la revendication 5 ou 6 ;
b2) amplifier ledit ARN ;
c2) détecter les produits d'amplification ;
d2) comparer les produits d'amplification obtenus à ceux obtenus avec un échantillon contrôle, et détecter de cette manière une éventuelle anomalie dans ledit transcrit du gène NPHS2, indicatrice d'un syndrome néphrotique cortico-résistant lié à une mutation du gène NPHS2.

Font également partie de l'invention les acides nucléiques isolés, consistant en une séquence différant de la séquence SEQ ID n° 1 par une mutation, insertion ou délétion, notamment dans l'une au moins des positions des nucléotides 481, 173/174, 488, 924/925, 128, 343, 482, 548, 607 et 940, ou encore 1033, 529, 622, 774-782, 154, 422, 442, 571, 572, 583, 783, 794 et 848; sous réserve que ledit acide nucléique isolé soit impliqué dans un syndrome néphrotique cortico-résistant.

Parmi les mutations déjà identifiées, on relève notamment les suivantes, présentées dans les tableaux 1 et 2.

**Tableau 1 : Mutations dans le gène NPHS2**

| Type de mutation^{a} | Changement de nucléotide | Effet sur la séquence codante | Exon | n° d'identification de la famille concernée | Statut de la mutation^{b} | Position sur SEQ ID n° 1 |
|---|---|---|---|---|---|---|
| Non-sens | C→T à 412 | R138X | 3 | 8 | H | 481 |
| Délétion/Insertion | Insertion de G à 104/5 | Décalage | 1 | 14 | h | 173/174 |
| | Délétion de G à 419 | Décalage | 3 | 14 | h^{c} | 488 |
| | Délétion de AA à 855/6 | Décalage | 7 | 9 | h | 924/925 |
| | C→T à 59 | P20L | 1 | 15 | H | 128 |
| Faux-sens | G→T à 274 | G92C | 1 | 3 | h^{c,d} | 343 |
| | G→A à 413 | R138Q | 3 | 4 | h^{c} | 482 |
| | | | | 6 | H | |
| | | | | 7 | H | |
| | | | | 11 | H | |
| | | | | 12 | h | |
| | | | | 13 | H | |
| | A→G à 479 | D160G | 4 | 16 | H | 548 |
| | G→A à 538 | V180M | 5 | 10 | H | 607 |
| | | | | 12 | h | 940 |
| | C→T à 871 | R291W | 7 | 2 | h^{c} | |

| | | | | | | |
|---|---|---|---|---|---|---|
| ^{a} : la position des mutations est indiquée en prenant pour base 1, le A du codon ATG et selon la nomenclature de Antonarakis et al ^{b} : H = mutation homozygote ; h = mutation hétérozygote ^{c} : uniquement la mutation paternelle détectée ^{d} : implique le dernier nucléotide de l'exon 1 et donc probablement altère aussi l'épissage | | | | | | |

Les mutations n'ont pas été retrouvées chez 40 contrôles

**Tableau 2 : Autres mutations dans le gène NPHS2**

| Type de Mutation | Changement de Nucléotide ^{a} | Effet sur la Séquence Codante | Exon | Nombre de familles | Statut de la Mutation ^{b} | Position sur SEQ ID n°1 |
|---|---|---|---|---|---|---|
| Non-sens | C→T à 964 | R322X | 8 | 1 | h | 1033 |
| | | | | | | |
| Délétion/Insertion | Insertion T à 460 | Décalage | 4 | 1 | h | 529 |
| | Déletion T 553 | Décalage | 5 | 1 | h | 622 |
| | Délétion 9pb à 705-713 | Délétion TER 236-238 | 5 | 1 | H | 774-782 |
| | | | | | | |
| Faux-sens | G→A à 85 | A29T | 1 | 1 | h | 154 |
| | C→T à 353 | P118L | 2 | 1 | h | 422 |
| | G→A à 373 | A125I | 2 | 1 | h | 442 |
| | C→A à 502 | R168S | 4 | 1 | h | 571 |
| | C→T à 502 | R168C | 4 | 1 | h | 571 |
| | G→A à 503 | R168H | 4 | 1 | h | 572 |
| | C→G à 514 | L172V | 4 | 1 | h | 583 |
| | G→T à 714 | R238S | 5 | 1 | h | 783 |
| | C→T à 725 | A242V | 5 | 2 | h | 794 |
| | T→A à 779 | V260E | 6 | 2 | H | 848 |

| | | | | | | |
|---|---|---|---|---|---|---|
| ^{a} la position des mutations est indiquée en prenant pour base 1, le A du codon ATG et selon la nomenclature de Antonarakis *et al.* ^{b} H = mutation homozygote; h = mutation hétérozygote. | | | | | | |

Les mutations n'ont pas été retrouvées chez 40 contrôles.

Ces tests peuvent être notamment mis à profit dans les familles qui ont déjà un enfant atteint, pour le diagnostic présymptomatique (en particulier, diagnostic prénatal).

Dans les cas sporadiques, la détection d'une mutation du gène NPHS2 permet de modifier le traitement (et en particulier d'éviter des traitements immunosuppresseurs qui seront inefficaces) et de prédire l'absence de récidive après transplantation rénale.

Ce test diagnostic peut également servir à rechercher l'association de certains variants polymorphes de la podocine dans d'autres pathologies impliquant secondairement des anomalies du filtre glomérulaire (néphropathie diabétique, néphropathie du SIDA, réduction néphronique, hypertension artérielle). Ces variants pourraient représenter des facteurs de susceptibilité au déclenchement ou à la progression de la néphropathie dans ces maladies.

L'invention a également pour objet des anticorps dirigés contre le polypeptide podocine tel que défini dans la revendication 2 ou 3.

Il peut s'agir d'anticorps poly- ou monoclonaux ou de leurs fragments, d'anticorps chimériques, notamment humanisés ou immunoconjugués.

Les anticorps polyclonaux peuvent être obtenus à partir du sérum d'un animal immunisé contre un polypeptide selon les modes opératoires usuels.

Selon un mode de réalisation de l'invention, on peut utiliser comme antigène un fragment peptidique approprié, pouvant être couplé par l'intermédiaire d'un résidu réactif à une protéine ou un autre peptide. Des lapins sont immunisés avec l'équivalent de 1mg de l'antigène peptidique selon la procédure décrite par Benoit *et al*. (1982). A des intervalles de quatre semaines, les animaux sont traités par des injections de 200 µg d'antigène et saignés 10 à 14 jours plus tard. Après la troisième injection, l'anti-sérum est examiné pour déterminer sa capacité à se lier au peptide antigène radiomarqué à l'iode, préparé par la méthode chloramine-T et est ensuite purifié par une chromatographie sur colonne échangeuse d'ion carboxyméthyl cellulose (CMC). Les molécules d'anticorps sont ensuite recueillies dans les mammifères et isolées jusqu'à la concentration souhaitée par les méthodes bien connues de l'homme de l'art, par exemple, en utilisant DEAE Sephadex pour obtenir la fraction IgG.

Afin d'augmenter la spécificité du sérum polyclonal, les anticorps peuvent être purifiés par une chromatographie d'immuno-affinité en utilisant des polypeptides immunisant en phase solide. L'anticorps est mis en contact avec le polypeptide immunisant en phase solide pendant une durée suffisante de façon à faire immuno-réagir le polypeptide avec la molécule d'anticorps afin de former un complexe immunologique en phase solide.

A titre d'exemple, des anticorps polyclonaux chez le lapin ont été produits contre deux protéines recombinantes comprenant les fragments des acides aminés 15 à 89 et 135 à 383 de la podocine, couplés à six résidus histidine du côté N-terminal, les ADNc ayant été sous-clonés dans le vecteur PQ E32 (Quiagen), et exprimés dans *E*. *coli.*

Des anticorps monoclonaux peuvent être obtenus selon la méthode classique de culture d'hybridomes décrite par Köhler et Milstein (1975).

Les anticorps ou fragments d'anticorps de l'invention peuvent être par exemple des anticorps chimériques, des anticorps humanisés, des fragments Fab et F(ab')2. Ils peuvent également se présenter sous forme d'immunoconjugués ou d'anticorps marqués.

Les anticorps de l'invention, en particulier les anticorps monoclinaux, peuvent notamment être utilisés pour l'analyse par immunohistochimie de la podocine sur des coupes de tissus spécifiques, par exemple par immunofluorescence, marquage à l'or, immunoperoxydase...

Les anticorps ainsi produits peuvent être avantageusement mis en oeuvre dans toute situation où l'expression de la podocine doit être observée.

L'invention a également pour objet l'utilisation d'au moins un anticorps selon la revendication 11 pour la détection ou la purification d'un polypeptide tel que défini dans la revendication 2 ou 3 dans un échantillon biologique.

Plus précisément, on décrit ici une méthode *in vitro* pour la détection ou la mesure du taux d'expression de la podocine dans un prélèvement biologique comprenant la mise en contact d'au moins un anticorps tel que défini précédemment avec ledit prélèvement biologique dans des conditions permettant la formation éventuelle de complexes immunologiques spécifiques entre la podocine et le ou lesdits anticorps et la détection des complexes immunologiques spécifiques éventuellement formés. La mise au point d'un tel test (de type ELlSA par exemple) pourrait être en particulier utile pour rechercher le développement d'anticorps anti-podocine après transplantation rénale, dans certaines maladies rénales auto-immunes, voire dans le syndrome néphrotique cortico-sensible.

On décrit également un kit pour la mise en oeuvre de cette méthode comprenant :
- au moins un anticorps spécifique de la podocine, éventuellement fixé sur un support ;
- des moyens de révélation de la formation de complexes antigènes/anticorps spécifiques entre la podocine et ledit anticorps et/ou des moyens de quantification de ces complexes.

L'invention a également pour objet une composition pharmaceutique comprenant un polypeptide podocine tel que défini dans la revendication 2 ou 3 ou un acide nucléique codant pour ledit polypeptide, en association avec un véhicule pharmaceutiquement acceptable.

Les modes d'administration, les posologies et les formes galéniques des compositions pharmaceutiques selon l'invention, contenant au moins un polypeptide, peuvent être déterminées de manière usuelle par l'homme du métier, notamment selon les critères généralement pris en compte pour l'établissement d'un traitement thérapeutique adapté à un patient, comme par exemple l'âge ou le poids corporel du patient, la gravité de son état général, la tolérance au traitement, et les effets secondaires constatés, etc.

De manière générale, une quantité thérapeutiquement ou prophylactiquement efficace variant d'environ 0,1 µg à environ 1 mg peut être administrée à des adultes humains.

On décrit également une composition pharmaceutique comprenant un acide nucléique tel que défini précédemment, codant pour un polypeptide à activité de podocine et un véhicule pharmaceutiquement acceptable, ladite composition étant destinée à être utilisée en thérapie génique. L'acide nucléique de préférence inséré dans un vecteur généralement viral (tels que les adénovirus et les rétrovirus) peut être administré sous forme nue, exempt de tout véhicule favorisant le transfert à la cellule cible, tels que des liposomes anioniques, des lipides cationiques, des microparticules, par exemple des microparticules d'or, des agents de précipitation, par exemple du phosphate de calcium, ou tout autre agent facilitant la transfection. Dans ce cas, le polynucléotide peut être simplement dilué dans une solution physiologiquement acceptable, telle qu'une solution stérile ou une solution stérile tampon, en présence ou en l'absence d'un véhicule.

De manière alternative, un acide nucléique de l'invention peut être associé à des agents qui facilitent la transfection. Il peut être, entre autres , (i) associé à un agent chimique qui modifie la perméabilité cellulaire tel que la bupivacaïne ; (ii) encapsulé dans des liposomes, éventuellement en présence de substances supplémentaires facilitant la transfection ; ou (iii) associé à des lipides cationiques ou des microparticules de silice, d'or ou de tungstène.

Lorsque les constructions d'acide nucléique de l'invention recouvrent des microparticules, celles-ci peuvent être injectées par voie intradermique ou intraépidermique par la technique du canon à gènes, "gene gun" (WO 94/24263).

La quantité à utiliser comme médicament dépend notamment de la construction d'acide nucléique elle-même, de l'individu auquel cet acide nucléique est administré, du mode d'administration et du type de formulation, et de la pathologie. De manière générale, une quantité thérapeutiquement ou prophylactiquement efficace variant d'environ 0,1 µg à environ 1 mg, de préférence d'environ 1 µg à environ 800 µg et, de manière préférentielle d'environ 25 µg à environ 250 µg, peut être administrée à des adultes humains.

Les constructions d'acides nucléiques de l'invention peuvent être administrées par toute voie d'administration conventionnelle telle que notamment par voie parentérale. Le choix de la voie d'administration dépend en particulier de la formulation choisie. Une administration ciblée au tissu rénal, en particulier aux glomérules, peut être particulièrement avantageuse.

Le polypeptide de l'invention ou l'acide nucléique codant pour ce polypeptide sont utiles à titre de médicament, notamment pour le traitement d'une maladie rénale, en particulier pour le traitement d'un syndrome néphrotique cortico-résistant lié à une mutation du gène NPHS2 ou survenant dans le cadre d'une maladie générale (SIDA, diabète...).

On décrit aussi une méthode de traitement thérapeutique, dans laquelle on administre à un patient nécessitant un tel traitement, une quantité efficace d'un polypeptide podocine tel que défini précédemment ou un acide nucléique codant pour ce polypeptide.

Le patient visé est généralement un être humain, mais l'application peut également être étendue à tout mammifère le cas échéant.

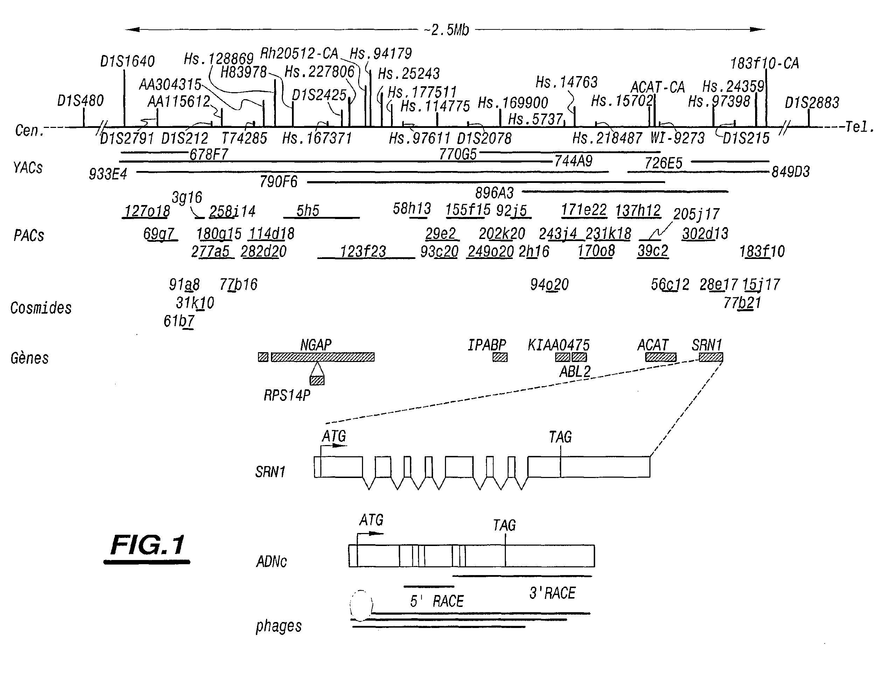

Les exemples suivants ainsi que la figure annexée illustrent l'invention sans en limiter sa portée.

### LEGENDE DE LA FIGURE

La figure annexée est une carte de la région NPHS2. La région candidate de 2,5 Mb est délimitée par les marqueurs *D1S1640* et *183f10-CA.* La position sur la carte des marqueurs polymorphes et des séquences STS (en caractères gras et italiques) et des séquences EST uniques et des clusters UniGene de EST (en caractère normal) est indiquée. Les YAC, PAC et cosmides sont représentés par des lignes. Les gènes sont indiqués par des cadres hachurés. *NGAP* est représenté par deux cadres séparés par une ligne horizontale qui symbolise la présence des exons épissés de manière alternative en position 5' probablement due à un promoteur alternatif. *RPS14P,* un pseudogène de la protéine ribosomale S14, est localisé à l'intérieur de l'intron de *NGAP.*

### EXEMPLES

### EXEMPLE 1 - Identification du gène NPHS2

L'approche utilisée par les auteurs de la présente invention pour identifier le gène NPHS2, a été de définir l'intervalle génétique minimal où se situait le gène, puis d'établir la carte physique de la région en construisant un contig de PAC couvrant la région, de faire l'inventaire des gènes connus et des EST de la région et de caractériser les EST (par RACE-PCR et criblage de banque d'ADNc de rein foetal).

### 1. Cartographie physique de la région candidate et localisation du gène NPHS2:

Une analyse de liaison à l'aide de marqueurs microsatellite (Dib et al., 1996) ainsi que de nouvelles familles de patients, a permis de localiser le locus NPHS2 entre les marqueurs D1S480 et D1S2883. Un contig de YAC (20 clones) couvrant la région entre ces deux marqueurs a été construit. Un contig de chromosomes artificiels P1 (PAC) a également été construit pour couvrir cette région estimée à environ 3 Mb. D'autres marqueurs microsatellites ont alors pu être caractérisés dans ce contig. Deux familles présentant des événements de recombinaison ont permis de localiser précisément le locus de la maladie entre D1S1640 et 183F10CA, nouveau marqueur microsatellite identifié par séquençage de sous-clones de la région. Le contig de 35 PAC entre ces deux marqueurs couvre environ 2 à 2,5 Mb, mais contient 5 trous partiellement remplis par 14 cosmides.

Les auteurs de l'invention ont alors localisé sur ce contig, en recherchant dans les banques de données les séquences potentiellement localisées dans la région et en séquençant les extrémités des YAC, des PAC et des cosmides ainsi que des sous-clones de différents PAC contenant potentiellement des îlots CpG, des gènes déjà connus, des clusters d'EST (UniGene) et des EST indépendants.

### 2. Identification du gène NPHS2

En consultant la base de données du Sanger Centre, il a été trouvé que le PAC 545A16 contenait le marqueur D1S215 localisé près de la borne télomérique de la région d'intérêt, ainsi que l'EST AA398634, qui provenait d'une banque de testicule et contenait de courtes séquences faiblement homologues au gène de la stomatine, mais curieusement *a priori* dans le sens opposé à l'EST. Les auteurs de l'invention ont alors localisé cet EST sur le cosmide 28e17 et sur le PAC 302d13 et montré par RT-PCR qu'il était exprimé dans le rein.

De multiples essais de RACE-PCR ont ensuite été nécessaires pour obtenir un ADNc à partir de cet EST. En effet, les produits obtenus correspondaient, dans la plupart des expériences, à des ADN génomiques qui paraissaient non épissés. Toutefois, l'un des produits obtenu correspondait à un transcrit contenant une courte phase ouverte de lecture et homologue à six EST (cluster Unigene Hs. 192657), provenant tous d'une banque de rein humain, mais qui n'avaient pas été localisés sur le génome. Il s'avère en fait que les EST du cluster Unigene Hs. 254975 auquel appartient l'EST AA398634 semblent appartenir à un autre gène ou pseudogène dont le sens de la transcription est opposé à NPHS2 et qui chevauche partiellement la séquence 3' de NPHS2, ce qui explique les données fournies par les banques de données concernant l'EST AA398634. En utilisant ce produit de RACE-PCR décrit ci-dessus comme sonde pour hybrider un Northern blot contenant des ARN de différents tissus, il a été montré que ce-transcrit d'environ 2 kb était exprimé seulement dans le rein. Ces résultats ont été confirmés en hybridant un dot blot contenant des ARN de 50 tissus différents (Clontech). Un fort signal a été obtenu seulement avec le rein adulte et le rein foetal. La localisation de ce gène sur le contig et son expression quasi-exclusive dans le rein faisaient de ce gène un excellent gène candidat, hypothèse renforcée par la quasi-absence de produit d'amplification par RT-PCR, en utilisant des amorces situées à la fois dans la partie 5' et dans la partie 3' de l'ADNc, avec l'ARN extrait du rein terminal d'un patient. L'ADNc complet du gène NPHS2 a été cloné par criblage d'une banque d'ADNc de rein foetal humain avec la sonde utilisée pour hybrider le Northern Blot (séquence ID n° 11).

Les jonctions introns-exons et les séquences génomiques en amont de l'exon 1 ont été obtenues par séquençage direct du PAC 302d13 et du cosmide 28e17.

### EXEMPLE 2 : Identification de mutations chez des familles de patients

Les auteurs de l'invention ayant caractérisé la structure intron-exon du gène, ils ont ensuite recherché par SSCP ("Single Strand Conformation Polymorphism") des mutations chez 16 patients non apparentés présentant un syndrome néphrotique cortico-résistant familial tel que décrit précédemment (début précoce, évolution rapide vers l'insuffisance rénale terminale, pas de récidive après transplantation et lésions de sclérose glomérulaire segmentaire et focale sur les biopsies rénales) et appartenant à des familles dans lesquelles l'étude des haplotypes était compatible avec une liaison au locus NPHS2.

Pour cette analyse SSCP, les exons ont été amplifiées par PCR en utilisant des amorces introniques flanquantes. Les conditions de PCR et les amorces ont été choisies en utilisant le programme Oligo 5.0 (NBI) et étaient les suivantes : à des températures d'hybridation de 50°C (exon 6), 55°C (exons 2, 3, 4 et 5), 60°C (exons 1, 7 et 8). En raison de la forte teneur en GC de l'exon 1, la PCR a été effectuée en utilisant la Taq polymérase de Qiagen et la solution -Q selon les instructions du fabricant. De plus, à cause de sa taille, le produit de PCR de l'exon 1 a dû être digéré par l'enzyme *Smal* en deux fragments avant l'électrophorèse sur gel. La migration a été menée pendant deux heures à 600 V, 25 mA et 15 W avec le Genephor Electrophoresis Unit, en utilisant le kit GeneGel Excel 12.5/24 (Pharmacia). La coloration a été réalisée avec un colorateur "GeneStain Automated Gel Stainer" en utilisant le kit PlusOne Silver Staining (Pharmacia).

### Résultats

Dix mutations différentes ont été observées. Certaines aboutissent à un décalage du cadre de lecture ou à l'apparition d'un codon stop prématuré et sont donc des mutations inactivatrices, ce qui prouve que le gène identifié est bien le gène NPHS2. D'autres sont des mutations faux-sens survenant dans des régions très conservées de la protéine, ségregeant dans les familles avec la maladie, et non retrouvées dans 80 chromosomes témoins, ce qui suggère fortement que ces mutations sont bien responsables du phénotype chez les enfants atteints.

Une des mutations faux-sens (R138Q) a été retrouvée chez six individus non apparentés mais provenant de la même partie de l'Europe, suggérant la possibilité d'un effet fondateur pour cette mutation.

### EXEMPLE 3 - Etude de l'expression du gène NPHS2 dans le rein par hybridation in situ

### Méthode

Des sections de rein de 6 µm recouvertes de paraffine ont été débarrassées de leur paraffine et réhydratées puis traitées aux micro-ondes dans du tampon de citrate de sodium (0,01 M, pH 6) pour augmenter le signal d'hybridation. Les ribosondes de NPHS2 ont été synthétisées à partir du produit de PCR de 1065 paires de bases (position 728 à 1792 de l'ADNc de NPHS2, SEQ ID n° 1) sous-cloné dans le vecteur PGEM-Teasy. La sonde antisens a été synthétisée après digestion avec *Sal*I en utilisant la polymérase T7 et la sonde sens après digestion avec *Sac*ll en utilisant la polymérase Sp6. Les ribosondes ont été marquées avec soit de la digoxigénine-11-UTP (Boehringer Mannheim) selon les instructions du fabricant ou avec la [³⁵S]UTP comme décrit dans Sibony et al, 1995. Des expériences d'hybridation *in situ* ont été réalisées comme décrit dans Kalatzis *et al*. (1998) et Heidet *et al*. (1997) pour des sondes de digoxigénine-11-UTP et [³⁵S]UTP, respectivement.

### Résultats

Ces expériences d'hybridation *in situ* ont permis de montrer que le gène NPHS2 était exprimé uniquement au niveau des podocytes dans le rein mature. Dans les reins foetaux, aucun signal n'a été observé aux stades précoces du développement du néphron. En revanche, des signaux intenses ont été détectés dans le segment inférieur du corps en S, dans la région correspondant aux futurs podocytes. Cette expression persiste dans les glomérules immatures et dans les glomérules matures du cortex profond. Ces résultats qui montrent l'expression exclusive du gène NPHS2 dans les podocytes, à la fois précocement au cours du développement et dans les glomérules matures, sont tout à fait en accord avec la pathologie observée et justifient la dénomination de "podocine" pour la protéine codée par le gène NPHS2.

### BIBLIOGRAPHIE

- Antonarakis S.E., Diagnosis of genetic disorders at the DNA level. N Engl J. Med. 320:153-163 (1989).
- Antonarakis, S.E., Recommendations for a nomenclature system for human gene mutations. Nomenclature Working Group. Hum. Mut. 11, 1-3 (1998).
- Bellis et al., medecine/sciences, 13:1317-24, (1997).
- Benoit et al., PNAS USA, 79, 917-921 (1982).
- Broyer M., Meyrier A., Niaudet P. & Habib R. Minimal changes and focal segmental glomerular sclerosis. In Oxford Textbook of Clinical Nephrology 2nd ed. (eds Davison A.M. et al.) 493-535 (Oxford University Press, Inc., 1998).
- Cooper et al., Diagnosis of genetic disease using recombinant DNA, 3rd Edition, Hum Genet., 87:519-560 (1991).
- Conton, P.J. et al., Clinical and pathologic features of familial focal segmental glomerulosclerosis. Am. J. Kidney Dis. 26, 34-40 (1995).
- Dib, C. et al. A comprehensive genetic map of the human genome based on 5,264 microsatellites. Nature 380, 152-154 (1996).
- Fuchshuber, A. et al. Mapping a gene (NPHS2) to chromosome 1 q25-q31 in idiopathic nephrotic syndrome confirms a distinct entity of autosomal recessive nephrosis. Hum. Mol. Genet. 4, 2155-2158 (1995).
- Heidet, L. et al. Diffuse leiomyomatosis associated with X-linked Alport syndrome: extracellular matrix study using immunohistochemistry and in situ hybridization. Lab. Invest. 76, 233-243 (1997).
- Huber, C.G. et al., Rapid and accurate sizing of DNA fragments by ion-pair chromatography on alkylated nonporous poly(styrenedivinylbenzene) particles. Anal. Chem. 67, 578-585 (1995).
- Kuklin, A. et al., Detection of single-nucleotide polymorphisms with the WAVE™ DNA fragment analysis system. Genetic Testing 1, 201-206 (1997/98).
- Kalatzis, V., Sahly, I., El-Amraoui, A. & Petit, C. Eya1 expression in the developing ear and kidney: towards the understanding of the pathogenesis of Branchio-Oto-Renal (BOR) syndrome. Dev. Dyn. 213, 486-499 (1998).
- Kestilä, M. et al. Positionally cloned gene for a novel glomerular protein-nephrin-is mutated in congenital nephrotic syndrome. Mol. Cell 1, 575-582 (1998)
- Köhler et Milstein, Nature, 256, 495-497, (1975).
- Lench et al., Am. J. Hum. Genet. 63, A296 (1998).
- Mathis, B.J. et al. A locus for inherited focal segmental glomerulosclerosis maps to chromosome 19q13. Kidney. Int. 53, 282-286 (1998).
- Sambrook et al., Molecular cloning, a laboratory manual Spring Harbor Laboratory Press, 9.54-62 (1989)
- Tsukaguchi et al., Adult onset familial FSGS mapping to chromosome 1 q. J. Am. Soc. Nephrol. 10, 443A (1999).
- Winn, M.P. et al. Linkage of a gene causing familial focal segmental glomerulosclerosis to chromosome 11 and further évidence of genetic heterogeneity. Genomics 58, 113-120 (1999).

## Revendications

1. Acide nucléique isolé, dont la séquence est choisie parmi SEQ ID n° 3 à SEQ ID n° 10, qui représente un fragment de l'ADN génomique du gène humain désigné NPHS2.

2. Polypeptide isolé, dénommé podocine, consistant en la séquence d'acides aminés SEQ ID n° 2.

3. Polypeptide isolé, consistant en une séquence homologue à la séquence d'acides aminés SEQ ID n°2, ladite séquence homologue étant définie comme :
i) une séquence identique à au moins 70% de la séquence SEQ ID n° 2; ou
ii) une séquence codée par une séquence d'acide nucléique hybridant avec la séquence SEQ ID n° 1 ou sa séquence complémentaire, dans des conditions stringentes d'hybridation, telles que fournies par une température d'hybridation inférieure de 5 à 30°C au Tm, et un tampon d'hybridation 6xSSC,
étant entendu que ledit polypeptide présente l'activité biologique de la podocine, polypeptide de séquence SEQ ID n°2.

4. Acide nucléique isolé, consistant en une séquence différant de la séquence SEQ ID n° 1 par une mutation, insertion ou délétion, dans au moins une position parmi les nucléotides 481, 173/174, 488, 924/925, 128, 343, 482, 548, 607 et 940, ou encore 1033, 529, 622, 774-782, 154, 422, 442, 571, 572, 583, 783, 794 et 848,
sous réserve que ledit acide nucléique isolé soit impliqué dans un syndrome néphrotique cortico-résistant.

5. Acide nucléique isolé, consistant en la séquence SEQ ID n° 1 codant pour un polypeptide désigné podocine.

6. Acide nucléique isolé, consistant en une séquence homologue de la séquence SEQ ID n°1, ladite séquence homologue étant définie comme :
i) une séquence identique à au moins 70 % de la séquence SEQ ID n° 1, étant entendu que ladite séquence code pour un polypeptide présentant l'activité biologique du polypeptide désigné podocine de séquence SEQ ID n°2; ou
ii) une séquence codant pour le polypeptide, tel que défini à la revendication 2 ou 3.

7. Vecteur de clonage et/ou d'expression contenant un acide nucléique selon l'une des revendications 1, 4, 5, ou 6.

8. Cellule hôte transfectée par un vecteur selon la revendication 7.

9. Acide nucléique constitué d'une séquence choisie parmi les séquences SEQ ID n° 11 à SEQ ID n° 27.

10. Procédé de production d'un polypeptide podocine recombinant, dans laquelle un vecteur contenant un acide nucléique selon la revendication 5 ou 6 est transféré dans une cellule hôte, qui est mise en culture dans des conditions permettant l'expression du polypeptide selon la revendication 2 ou 3 respectivement.

11. Anticorps dirigé contre le polypeptide tel que défini dans la revendication 2 ou 3.

12. Utilisation d'au moins un anticorps selon la revendication 11 pour la détection ou la purification d'un polypeptide tel que défini dans la revendication 2 ou 3 dans un échantillon biologique.

13. Utilisation d'au moins un acide nucléique selon la revendication 1, 4, 5, ou 6, pour la détection d'une anomalie dans le gène NPHS2, défini comme comprenant une séquence d'acide nucléique selon la revendication 1 ou, dans son transcrit, défini comme comprenant une séquence d'acide nucléique de la revendication 5 ou 6.

14. Procédé de diagnostic *in vitro* d'un syndrome néphrotique cortico-résistant, comprenant les étapes consistant à :
a1) mettre en présence un échantillon biologique contenant de l'ADN avec des oligonucléotides spécifiques permettant l'amplification de tout ou partie du gène NPHS2, défini comme comprenant une séquence d'acide nucléique selon la revendication 1;
b1) amplifier ledit ADN ;
c1) détecter les produits d'amplification ;
d1) comparer les produits d'amplification obtenus à ceux obtenus avec un échantillon contrôle, et détecter de cette manière une éventuelle anomalie dans ledit gène NPHS2, indicatrice d'un syndrome néphrotique cortico-résistant ;
ou, selon une alternative,
a2) mettre en présence un échantillon biologique contenant de l'ARN avec des oligonucléotides spécifiques permettant l'amplification de tout ou partie du transcrit du gène NPHS2, défini comme comprenant une séquence d'acide nucléique de la revendication 5 ou 6 ;
b2) amplifier ledit ARN ;
c2) détecter les produits d'amplification ;
d2) comparer les produits d'amplification obtenus à ceux obtenus avec un échantillon contrôle, et détecter de cette manière une éventuelle anomalie dans ledit transcrit du gène NPHS2, indicatrice d'un syndrome néphrotique cortico-résistant.

15. Composition pharmaceutique comprenant un polypeptide selon la revendication 2 ou 3, ou un acide nucléique codant pour ledit polypeptide, en association avec un véhicule pharmaceutiquement acceptable.

16. Polypeptide selon la revendication 2 ou 3, ou un acide nucléique codant pour ledit polypeptide, pour son utilisation dans le traitement de maladies rénales, notamment d'un syndrome néphrotique cortico-résistant.

## Claims

1. Isolated nucleic acid, the sequence of which is chosen from SEQ ID No. 3 to SEQ ID No. 10, which represents a fragment of the genomic DNA of the human gene designated NPHS2.

2. Isolated polypeptide, called podocin, consisting of the amino acid sequence SEQ ID No. 2.

3. Isolated polypeptide, consisting of a sequence homologous to the amino acid sequence SEQ ID No. 2, the said homologous sequence being defined as:
i) a sequence identical to at least 70 % of the sequence SEQ ID No. 2; or
ii) a sequence coded by a nucleic acid sequence which hybridizes with the sequence SEQ ID No. 1 or its complementary sequence under stringent hybridization conditions such as are provided by a hybridization temperature 5 to 30°C lower than the Tm and a 6xSSC hybridization buffer.
it being understood that the said polypeptide has the biological activity of podocin, the polypeptide of SEQ ID No. 2.

4. Isolated nucleic acid consisting of a sequence which differs from the sequence SEQ ID No. 1 by a mutation, insertion or deletion in at least one position among nucleotides 481, 173/174, 488, 924/925, 128, 343, 482, 548, 607 and 940, or 1033, 529, 622, 774-782, 154, 422, 442, 571, 572, 583, 783, 794 and 848, with the proviso that the said isolated nucleic acid is involved in a corticoresistant nephrotic syndrome.

5. Isolated nucleic acid consisting of the sequence SEQ ID No. 1 coding for a polypeptide called podocin.

6. Isolated nucleic acid consisting of a sequence homologous to the sequence SEQ ID No. 1, the said homologous sequence being defined as:
i) a sequence identical to at least 70 % of the sequence SEQ ID No. 1, it being understood that the said sequence codes for a polypeptide having the biological activity of the polypeptide called podocin, of sequence SEQ ID No. 2; or
ii) a sequence coding for the polypeptide as defined in claim 2 or 3.

7. Cloning and/or expression vector containing a nucleic acid according to any one of claims 1, 4, 5 or 6.

8. Host cell transfected by a vector according to claim 7.

9. Nucleic acid made up of a sequence chosen from the sequences SEQ ID No. 11 to SEQ ID No. 27.

10. Process for the production of a recombinant podocin polypeptide, in which a vector containing a nucleic acid according to claim 5 or 6 is transferred into a host cell, which is cultured under conditions which allow expression of the polypeptide according to claim 2 or 3 respectively.

11. Antibody directed against the polypeptide as defined in claim 2 or 3.

12. Use of at least one antibody according to claim 11 for the detection or purification of a polypeptide as defined in claim 2 or 3 in a biological sample.

13. Use of at least one nucleic acid according to claim 1, 4, 5 or 6 for the detection of an anomaly in the gene NPHS2, defined as comprising a nucleic acid sequence according to claim 1 or, in its transcript, defined as comprising a nucleic acid sequence of claim 5 or 6.

14. Method for the *in vitro* diagnosis of a corticoresistant nephrotic syndrome, comprising the stages consisting of:
a1) bringing a biological sample containing DNA into contact with specific oligonucleotides which allow amplification of all or part of the gene NPHS2,
defined as comprising a nucleic acid sequence according to claim 1;
b1) amplification of the said DNA;
c1) detection of the amplification products;
d1) comparison of the amplification products obtained with those obtained with a control sample, and
detection in this manner of any anomaly in the said gene NPHS2 indicating a corticoresistant nephrotic syndrome;
or, according to an alternative,
a2) bring a biological sample containing RNA into contact with specific oligonucleotides which allow amplification of all or part of the transcript of the gene NPHS2, defined as comprising a nucleic acid sequence according to claim 5 or 6;
b2) amplification of the said RNA;
c2) detection of the amplification products;
d2) comparison of the amplification products obtained with those obtained with a control sample, and detection in this manner of any anomaly in the said transcript of the gene NPHS2 indicating a corticoresistant nephrotic syndrome.

15. Pharmaceutical composition comprising a polypeptide according to claim 2 or 3, or a nucleic acid coding for the said polypeptide, in combination with a pharmaceutically acceptable vehicle.

16. Polypeptide according to claim 2 or 3, or a nucleic acid coding for the said polypeptide, for its use in the treatment of kidney diseases, in particular a corticoresistant nephrotic syndrome.

## Patentansprüche

1. Isolierte Nucelinsäure, deren Sequenz ausgewählt ist aus SEQ ID No. 3 bis SEQ ID No. 10, welche ein Fragment der genomischen DNA des als NPHS2 bezeichneten menschlichen Gens darstellt.

2. Isoliertes Polypeptid, welches als Podocin bezeichnet wird, bestehend aus der Aminosäuresequenz SEQ ID No. 2.

3. Isoliertes Polypeptid, welches aus einer zu der Aminosäuresequenz SEQ ID No. 2 homologen Sequenz besteht, wobei die homologe Sequenz definiert ist als:
i) eine Sequenz, die zu mindestens 70 % mit der Sequenz SEQ ID No. 2 identisch ist; oder
ii) eine Sequenz, die durch eine Nucleinsäuresequenz codiert wird, welche mit der Sequenz SEQ ID No. 1 oder ihrer komplementären Sequenz hybridisiert bei stringenten Hybridisierungsbedingungen, wie sie sich durch eine Hybridisierungstemperatur von weniger als 5 bis 30 °C bis Tm und einem Hybridisierungspuffer 6xSSC ergeben,
mit der Maßgabe, dass das Polypeptid die biologische Aktivität von Podocin, dem Polypeptid der Sequenz SEQ ID No. 2 aufweist.

4. Isolierte Nucleinsäure, bestehend aus einer Sequenz, die sich von der Sequenz SEQ ID No. 1 durch eine Mutation, Insertion oder Deletion in mindestens einer Position der Nucleotide 481, 173/174, 488, 924/925, 128, 343, 482, 548, 607 und 940 oder auch 1033, 529, 622, 774-782, 154, 422, 442, 571, 572, 583, 783, 794 und 848 unterscheidet, mit der Einschränkung, dass die isolierte Nucleinsäure an einem cortico-resistenten nephrotischen Syndrom beteiligt ist.

5. Isolierte Nucleinsäure, bestehend aus der Sequenz SEQ ID No. 1: codierend für ein als Podocin bezeichnetes Polypeptid.

6. Isolierte Nucleinsäure, bestehend aus einer zu der Sequenz SEQ ID No. 1 homologen Sequenz, wobei die homologe Sequenz definiert ist als:
i) eine Sequenz, die zu mindestens 70 % mit der Sequenz SEQ ID No. 1 identisch ist mit der Maßgabe, dass diese Sequenz für ein Polypeptid codiert, welches die biologische Aktivität des als Podocin bezeichneten Polypeptids der Sequenz SEQ ID No. 2 aufweist; oder
ii) eine Sequenz, die für das Polypeptid, wie es in Anspruch 2 oder 3 definiert worden ist, codiert.

7. Klonierungs- und/oder Expressionsvektor, enthaltend eine Nucleinsäure, nach einem der Ansprüche 1, 4, 5 oder 6.

8. Durch einen Vektor nach Anspruch 7 transfektierte Wirtszelle.

9. Nucleinsäure, bestehend aus einer Sequenz ausgewählt aus den Sequenzen SEQ ID No. 11 bis SEQ ID No. 27.

10. Verfahren zur Herstellung eines rekombinanten Podocin-Polypeptids, gemäß dem ein eine Nucleinsäure nach Anspruch 5 oder 6 enthaltender Vektor in eine Wirtszelle transferiert wird, die unter den Bedingungen, welche di3 Expression des Polypeptids nach Anspruch 2 bzw. 3 ermöglichen, gezüchtet wird.

11. Antikörper gegen das Polypeptid, wie es in Anspruch 2 oder 3 definiert worden ist.

12. Verwendung mindestens eines Antikörpers nach Anspruch 11 für den Nachweis oder die Reinigung eines Polypeptids, wie es in Anspruch 2 oder 3 definiert worden ist, in oder aus einer biologischen Probe.

13. Verwendung mindestens einer Nucleinsäure nach Anspruch 1, 4, 5 oder 6 zum Nachweis einer Anomalie in dem Gen NPHS2, das **dadurch** definiert ist, dass es eine Nucleinsäuresequenz nach Anspruch 1 umfasst, oder in seinem Transkriptionsprodukt, **dadurch** definiert, dass es eine Nucleinsäuresequenz nach Anspruch 5 oder 6 umfasst.

14. in vitro-Verfahren zur Diagnose eines cortico-resistenten nephrotischen Syndroms, umfassend die Schritte:
a1) Inkontaktbringen einer DNA enthaltenden biologischen Probe mit spezifischen Oligonucleotiden, welche die Amplifizierung des ganzen oder eines Teils des Gens NPHS2 ermöglichen, welches dahingehend definiert ist, dass es eine Nucleinsäuresequenz nach Anspruch 1 umfasst;
b1) Amplifizierung der DNA;
c1) Detektion der Amplifizierungsprodukte;
d1) Vergleich der erhaltenen Amplifizierungsprodukte mit jenen, die man mit einer Kontrollprobe erhalten hat, und in dieser Weise Nachweis einer eventuellen Anomalie in dem Gen NPHS2, die ein cortico-resistantes nephrotisches Syndrom anzeigt;
oder, gemäß einer Alternative,
a2) Inkontaktbringen einer RNA enthaltenden biologischen Probe mit Oligonucleotiden, welche die Amplifizierung des ganzen oder eines Teils des Transkriptionsprodukts des Gens NPHS2 ermöglichen, welches **dadurch** definiert ist, dass es eine Nucleinsäuresequenz nach Anspruch 5 oder 6 umfasst:
b2) Amplifizierung der RNA;
c2) Detektion der Amplifizierungsprodukte;
d2) Vergleich der erhaltenen Amplifizierungsprodukte mit jenen, die man mit einer Kontrollprobe erhalten hat, und in dieser Weise Nachweis einer eventuellen Anomalie in dem Transkriptionsprodukt des Gens NPHS2, die ein corticoresistentes nephrotisches Syndrom anzeigt.

15. Pharmazeutische Zubereitung, umfassend ein Polypeptid nach Anspruch 2 oder 3 oder eine Nucleinsäure, welche für das Polypeptid codiert, in Kombination mit einem pharmazeutisch annehmbaren Hilfsstoff.

16. Polypeptid nach Anspruch 2 oder 3 oder eine für das Polypeptid codierende Nucleinsäure, für die Verwendung bei der Behandlung von Nierenerkrankungen, insbesondere eines cortico-resistenten nephrotischen Syndroms.


### LISTE DE SEQUENCES

<110> INSERM
<120> Gène NPHS2 impliqué dans le syndrome néphrotique cortico-résistant, protéine codée par ce gène et utilisations diagnostiques et thérapeutiques
<130> BFF 99/0667
<140>
   <141>
<150> FR 0000709
   <151> 2000-01-20
<160> 29
<170> PatentIn Ver. 2.1
<210> 1
   <211> 1853
   <212> ADN
   <213> Homo sapiens
<220>
   <221> CDS
   <222> (70) .. (1221)
<400> 1
cacagggact gcgctccctt gcccctagcg ctcccgcgct gctgctccag ccgcccggca 60
   gctctgagg atg gag agg agg gcg cgg agc tcc tcc agg gag tcc cgc ggg 111
   cga ggc ggc agg act ccg cac aag gag aac aag agg gca aag gcc gag   159
   agg agc ggc ggg ggc cgc ggg cgc cag gag gct ggg ccc gag ccg tcg   207
   ggc tcc gga cgg gcg ggg acc ccg ggg gag ccc cga gcg ccc gcc gcc   255
   acg gtg gtg gac gtg gat gag gtc cga ggc tcc ggc gag gag ggc acc   303
   gag gtg gtg gcg ctg ttg gag agc gag cgg ccc gag gaa ggt acc aaa   351
   tcc tcc ggc tta ggg gcc tgt gag tgg ctt ctt gtc ctc att tcc ctg   399
   ctc ttc atc atc atg acc ttc cct ttt tcc atc tgg ttc tgc gta aag     447
   gtt gta caa gag tat gaa aga gta att ata ttc cga ctg gga cat ctg     495
   ctt cct gga aga gcc aaa ggc cct ggt ctt ttc ttt ttt ttg ccc tgc     543
   ctg gat acc tac cac aag gtt gac ctt cgt ctc caa act ctg gag ata     591
   cct ttt cat gag atc gtg acc aaa gac atg ttt ata atg gag ata gat     639
   gcc att tgc tac tac cga atg gaa aat gcc tct ctt ctc cta agc agt     687
   ctt gct cat gta tct aaa gct gtg caa ttc ctt gtg caa acc act atg     735
   aag cgt ctc cta gca cat cga tcc ctc act gaa att ctt cta gag agg     783
   aag agc atc gcc caa gat gca aag gtt gcc ttg gat tca gtg acc tgt     831
   att tgg gga atc aaa gtg gag aga ata gaa att aaa gat gtg agg ttg     879
   cca gct ggg ctt cag cac tca ctg gct gtg gag gct gaa gcg caa aga     927
   caa gcc aaa gtg cgg atg att gct gca gaa gcg gaa aag gct gct tct     975
   gag tcc ctg agg atg gca gct gag att ctg tca ggc acc cct gct gct     1023
   gtt cag ctt cga tac ctc cac acc ctt cag tct ctg tcc aca gag aag     1071
   cct tcc act gtg gtt tta cct ttg cca ttt gac cta ctg aat tgc ctg     1119
   tct tct ccc agc aac aga act cag gga agc ctc ccc ttc cca agt cct   1167
   tcc aaa cct gtt gag cca cta aat cct aaa aag aaa gac tct ccc atg   1215
   tta tag gaaggatggg gcataatgtg actgtaaagg ggcctgccat agaaaagtca   1271
   catccctgag ggagacactc tgtcctcatt ccctgccctt cctttggttg ccatatggaa 1331
   tggccatgga atgcacgaag tcacaatgca ccatccatga gaagacrgtg aaatgatgta 1391
   atgacagaga aggcagacaa catgtttccg tgactcatct agtcagagca attatgggaa 1451
   acagctttgg tcaacattct actttggaaa gaattttgag tctagatgtg gttaaatttt 1511
   gacttctggg aacttggttc agatgtccct ttcactgtat gtcctctgac ccctttggca 1571
   aggttgccac agctcccaca gcccttccta caagcaccta tcattgggct tgtcacactc 1631
   tattgctctt ctgtcccgaa gatgcagtct tctctccaat gatactacca agtcttagtt 1691
   ttcctcaacc acactcaatc tttttgctcc accctgaatt cctcacacct aaccctgata 1751
   gttacctaaa gtgacactta aatgtttcag agtgaatgca aaaaagagag atgtacttgg 1811
   agtcggatat acaatttatc cctaattaaa gcatttaaaa gg                    1853
<210> 2
   <211> 383
   <212> PRT
   <213> Homo sapiens
<400> 2
<210> 3
   <211> 1104
   <212> ADN
   <213> Homo sapiens
<400> 3
   aggatataaa ataagaaata cgtagggagg agagaaaggc atccttgaga cgactccaag 60
   aaggaaagtt ggggatgagg cgaaatttct gattttacct taaagtgacc ctaattcgat 120
   gaccttttgt ggtttttttc tttttctttt tttacttggc cctgcccaag caggacctaa 180
   aaacaaacag acaaaaaagg ttactaacaa ctgttcctct ccacgaaaat ctgcagtaaa 240
   aggtaaaaga tgtattcgtt ttgaagagaa accagagctt gcgatgagct tctgtatctc 300
   cgtcagccct ctagcatgac attaggaacc ctccaggaga tgagtcttca cagcccgggt 360
   tggcacctgc agacacgcac ttttcaacgc ccgcaccctg cccggggccg gctctcccac 420
   ccaggcctct ctctgcttca gcgccgcccc ggccgtggga gtcggcgggc gcagtccaca 480
   gctccaccaa gacacagctg tcggggttcc gggtgcgccc cgcccgcggc cccggtgtcc 540
   cgcccctcgc cctcagcccc cacccgacgg tctttagggt cccccgggca cgccacgcgg 600
   acccgcagcg actccacagg gactgcgctc ccgtgcccct agcgctcccg cgctgctgct 660
   ccagccgccc ggcagctctg aggatggaga ggagggcgcg gagctcctcc agggagtccc 720
   gcgggcgagg cggcaggact ccgcacaagg agaacaagag ggcaaaggcc gagaggagcg 780
   gcgggggccg cgggcgccag gaggctgggc ccgagccgtc gggctccgga cgggcgggga 840
   ccccggggga gccccgagcg cccgccgcca cggtggtgga cgtggattag gtccgaggct 900
   ccggcgagga gggcaccgag gtggtggcgc tgttggagag cgagcggccc gaggaaggta 960
   cggattcagc accactatct gctacttttc caggtggtaa ctaaggggcg tcagataagg 1020
   tggaaagggt catccccacg agacccactg aagccagagc agattgctgg atgctcaggt 1080
   tcccaggaac ggaagggcgt aagt 1104
<210> 4
   <211> 378
   <212> ADN
   <213> Homo sapiens
<400> 4
   tctgactact ctgatttgac ttattcctaa tatttccagc aaagtctcca agytgtgcca 60
   actccaatac caagaattgg accaacagat gctagtcagt gaatacagtg aagtttcaat 120
   ataattattg gtttgcttta atttttttaa ggtaccaaat cctccggctt aggggcctgt 180
   gagtggcttc ttgtcctcat ttccctgctc ttcatcatca tgaccttccc tttttccatc 240
   tggttctgcg taaaggtgag attccataag gacccaatag gtaatttcct gaggcctctc 300
   actggccaca ccatgcccat tctcacttct gttttctggt acatgttatt gctccatgtg 360
   gaatgccctc accccaga 378
<210> 5
   <211> 700
   <212> ADN
   <213> Homo sapiens
<400> 5
   catataaaag ctagtgcaga actcacagaa aatataagat ttaatatgcc ttgataagat 60
   taatttaggg aaagttggcc atggatttta gataatcata agtctttaat caaaattctg 120
   tctatgggtt caaaaattaa catggttaat atactttttc atttctgaaa ttttacactt 180
   actaaatata gattttggaa acttaagtat taatagaaat tttttcctgg ttctcaaaac 240
   aaaaaatttc tgatatctag gatcattctt atgccaaggc cttttgaaga ctttttcttt 300
   ctgggagtga tttgaaagga ttaaatttct ctttaggttg tacaagagta tgaaagagta 360
   attatattcc gactgggaca tctgcttcct ggaagagcca aaggccctgg taaaaaaaca 420
   ctcttttttt tctaaacacc tctctcctga cttgccaatt tcttcaaccc atgcagattt 480
   gtaatatgga cctcagatta aatgaagtaa cttgattcat gatatctgaa ttttccaatc 540
   tgttacttat aggttattca aatattcttc agagactatt actactaggt cataggtagc 600
   caagagagag aattggtaca gagagcccac atgccagggc aaggcttgct ggaatagcaa 660
   gttagcttag gaccaatggc tggggactga tgtgagtacg 700
<210> 6
   <211> 360
   <212> ADN
   <213> Homo sapiens
<400> 6
   ctggattaca tattataata tataatagtg        ctctcctttt accctcaggt ggaggtggga 60
   tgggccaatg gtctgtaatt agaggctaag aaaagtaatg tagtgtgcaa cctgacccca 120
   gaaaggtgaa acccaaacag cyttcatgct agctatttat cygtcayttc ctcctcctct 180
   cttttaggtc ttttcttttt tttgccctgc ctggatacct accacaaggt tgaccttcgt 240
   ctccaaactc tggagatacc ttttcatgag gtaagccaaa tgatggcttt tgctttctct 300
   atacattttc catggtctac ctaccgngga caaaatgatt atttatactc aaaaatagga 360
<210> 7
   <211> 649
   <212> ADN
   <213> Homo sapiens
<400> 7
   cttcttatga gaagattatt tctgattttt tttacaaaag gatttaccac aggattaagt 60
   tgtgcattct ttcgtgtatt taataaaaat ttcataattt tcaaaaacat gtctatttta 120
   aataaagggt aggccaactc catttttctc ttgcggagaa aattcacttt gaacacattt 180
   agttcctcta accccacata ggaaaggagc ccaagaatca agcctgtcat ccaaactttt 240
   ttctgcctag atcgtgacca aagacatgtt tataatggag atagatgcca tttgctacta 300
   ccgaatggaa aatgcctctc ttctcctaag cagtcttgct catgtatcta aagctgtgca 360
   attccttgtg caaaccacta tgaagcgtct cctagcacat cgatccctca ctgaaattct 420
   tctagagagg aagagcatcg cccaagatgc aaaggtactt agataaacat aatggccaat 480
   atgctgaaat atttatcttt tattcatttg ttcgttggac atttattaaa tcttctatgg 540
   ccagttccat cccttagggg ccatcccttt gggagctcat agctagttag gaggttgcca 600
   aattgactct gagtcaatta tagttatcag tatggtgctt gttaatcag             649
<210> 8
   <211> 592
   <212> ADN
   <213> Homo sapiens
<400> 8
   aattacattt agggccacct ggataatcca ggataatatc cccatctcaa gagcctttaa 60
   cctaatcaaa ccagcaaaat ccctcttggg gtaacattca cagattccag ggattaggac 120
   atgggatatc tttggggacc attattcagc ataccacacc atcttcaatt gcacagatat 180
   ttattgggtg gcaccatgca agttaaacaa ctctttgcaa ggcactgtga agttaaatac 240
   aacaggcaaa taatgtcctt tcaaagggaa tgttgttcct tagtacagaa caatggccac 300
   cagggtttag gcatgctctc ctcccacctg gaggctccca ctgacatctg aattcttctt 360
   tccacaggtt gccttggatt cagtgacctg tatttgggga atcaaagtgg agagaataga 420
   aatgtgggta ggaaattaac tagcaagaac tgtatgataa aggaaaatat tctggtttca 480
   ttttaaattt ttcatttgaa aaattatttt cactgagtac tatagccata tcagcataaa 540
   tttataaaaa agagaaacaa atcacctaat atcttacagc cataacacaa tc         592
<210> 9
   <211> 716
   <212> ADN
   <213> Homo sapiens
<400> 9
   cattgttcaa atttattagt tggggcttag attatatcct aagcggaaaa actgagcaca 60
   gctcatcaaa tacaaaacct gctgtgctga taatgagaaa ctacagctct actgtagcat 120
   cagcaataat acaaaactgc atttgaggca tcgaccttgg agatctgcct acttttgacc 180
   tcagaagtct aggaatggca cactctggtc actccaaatt tgctactcat catgagacag 240
   cagtagagag gcttgcaagt ctgtgtgaaa gctttggccc ctaaatcatg gctgcacacc 300
   tacatacctg cattctttct ttttcagtaa agatgtgagg ttgccagctg ggcttcagca 360
   ctcactggct gtggaggctg aagcgcaaag acaagccaaa gtgcgggtga gcactccatc 420
   ctcccaccca gactgccctt taggaaggcc tgctcgtgga gaacatttcc cctttgcttc 480
   cttactgtcc attcattagg cactgggcan aagctgtctt gggcccttac aactctatta 540
   aaattgctct cttaaagtgt gttaatagtc ccctgactaa tgcaactcct ctccctctct 600
   gaagctactg ataatagtga ccactcactg cttgagtctc accttccctc tctctcctta 660
   aaggcatctc ctccacacac atcaatccct cttctctagt gctggcatct tttcct     716
<210> 10
   <211> 1305
   <212> ADN
   <213> Homo sapiens
<400> 10
   ggcaaaatcc taatctttca aggcccacca gatgctaata actcccctaa tacttcattt 60
   atacttgtga tggctcctaa cgcattccac cttaaattgt gattaacagt ttaatctgtc 120
   tccccagctc aagacccttc agaaagaaga ataaacatgt tctatgctta accgtgcttg 180
   ccacatagta gatgctcagt gcttgtctgc tgagtcatac tgcatagtgg tgaagccttc 240
   agggaatgaa gaacaatcac tttgctttcg tcacatgttt tctagatgat tgctgcagaa 300
   gcggaaaagg ctgcttctga gtccctgagg atggcagctg agattctgtc aggcacccct 360
   gctgctgttc agcttcgata cctccacacc cttcagtctc tgtccacaga gaagccttcc 420
   actgtggttt tacctttgcc atttgaccta ctgaattgcc tgtcttctcc cagcaacaga 480
   actcagggaa gcctcccctt cccaagtcct tccaaacctg ttgagccact aaatcctaaa 540
   aagaaagact ctcccatgtt ataggaagga tggggcataa tgtgactgta aaggggcctg 600
   ccatagaaaa gtcacatccc tgagggagac actctgtcct cattccctgc ccttcctttg 660
   gttgccatat ggaatggcca tggaatgcac gaagtcacaa tgcaccatcc atgagaagac 720
   rgtgaaatga tgtaatgaca gagaaggcag acaacatgtt tccgtgactc atctagtcag 780
   agcaattatg ggaaacagct ttggtcaaca ttctactttg graagaattt tgagtctaga 840
   tgtggttaaa ttttgacttc tgggaacttg gttcagatgt ccctttcact gtatgtcctc 900
   tgaccccttt ggcaaggttg ccacagctcc cacagccctt cctacaagca cctatcattg 960
   ggcttgtcac actctattgc tcttctgtcc craagatgca gtcttctctc caatgatact 1020
   accaagtctt agttttcctc aaccacactc aatctttttg ctccaccctg aattcctcac 1080
   acctaaccct gatagttacc taaagtgaca cttaaatgtt tcagagtgaa tgcaaaaaag 1140
   agagatgtac ttggagtcgg atatacaatt tatccctaat taaagcattt aaaaggaatt 1200
   ctttttgtgg agattccttt tttaaacaaa taaataaaag gacaaaaaca tctgacacat 1260
   gtggcttaaa atctgaggga gaatcactat aaatagtggg ccaga                1305
<210> 11
   <211> 1065
   <212> ADN
   <213> Homo sapiens
<400> 11
   ccactatgaa gcgtctccta gcacatcgat ccctcactga aattcttcta gagaggaaga 60
   gcatcgccca agatgcaaag gttgccttgg attcagtgac ctgtatttgg ggaatcaaag 120
   tggagagaat agaaattaaa gatgtgaggt tgccagctgg gcttcagcac tcactggctg 180
   tggaggctga agcgcaaaga caagccaaag tgcggatgat tgctgcagaa gcggaaaagg 240
   ctgcttctga gtccctgagg atggcagctg agattctgtc aggcacccct gctgctgttc 300
   agcttcgata cctccacacc cttcagtctc tgtccacaga gaagccttcc actgtggttt 360
   tacctttgcc atttgaccta ctgaattgcc tgtcttctcc cagcaacaga actcagggaa 420
   gcctcccctt cccaagtcct tccaaacctg ttgagccact aaatcctaaa aagaaagact 480
   ctcccatgtt ataggaagga tggggcataa tgtgactgta aaggggcctg ccatagaaaa 540
   gtcacatccc tgagggagac actctgtcct cattccctgc ccttcctttg gttgccatat 600
   ggaatggcca tggaatgcac gaagtcacaa tgcaccatcc atgagaagac agtgaaatga 660
   tgtaatgaca gagaaggcag acaacatgtt tccgtgactc atctagtcag agcaattatg 720
   ggaaacagct ttggtcaaca ttctactttg gaaagaattt tgagtctaga tgtggttaaa 780
   ttttgacttc tgggaacttg gttcagatgt ccctttcact gtatgtcctc tgaccccttt 840
   ggcaaggttg ccacagctcc cacagccctt cctacaagca cctatcattg ggcttgtcac 900
   actctattgc tcttctgtcc cgaagatgca gtcttctctc caatgatact accaagtctt 960
   agttttcctc aaccacactc aatctttttg ctccaccctg aattcctcac acctaaccct 1020
   gatagttacc taaagtgaca cttaaatgtt tcagagtgaa tgcaa                1065
<210> 12
   <211> 20
   <212> ADN
   <213> Homo sapiens
<400> 12
   gcagcgactc cacagggact 20
<210> 13
   <211> 20
   <212> ADN
   <213> Homo sapiens
<400> 13
   tcagtgggtc tcgtggggat 20
<210> 14
   <211> 20
   <212> ADN
   <213> Homo sapiens
<400> 14
   aggcagtgaa tacagtgaag 20
<210> 15
   <211> 19
   <212> ADN
   <213> Homo sapiens
<400> 15
   ggcctcagga aattaccta 19
<210> 16
   <211> 20
   <212> ADN
   <213> Homo sapiens
<400> 16
   ttctgggagt gatttgaaag 20
<210> 17
   <211> 20
   <212> ADN
   <213> Homo sapiens
<400> 17
   tgaagaaatt ggcaagtcag 20
<210> 18
   <211> 19
   <212> ADN
   <213> Homo sapiens
<400> 18
   aaggtgaaac ccaaacagc 19
<210> 19
   <211> 20
   <212> ADN
   <213> Homo sapiens
<400> 19
   cggtaggtag accatggaaa 20
<210> 20
   <211> 20
   <212> ADN
   <213> Homo sapiens
<400> 20
   cataggaaag gagcccaaga 20
<210> 21
   <211> 20
   <212> ADN
   <213> Homo sapiens
<400> 21
   tttcagcata ttggccatta 20
<210> 22
   <211> 17
   <212> ADN
   <213> Homo sapiens
<400> 22
   ctcccactga catctga 17
<210> 23
   <211> 20
   <212> ADN
   <213> Homo sapiens
<400> 23
   aatttaaaat gaaaccagaa 20
<210> 24
   <211> 20
   <212> ADN
   <213> Homo sapiens
<400> 24
   ctaaatcatg gctgcacacc 20
<210> 25
   <211> 20
   <212> ADN
   <213> Homo sapiens
<400> 25
   cttcctaaag ggcagtctgg 20
<210> 26
   <211> 20
   <212> ADN
   <213> Homo sapiens
<400> 26
   ggtgaagcct tcagggaatg 20
<210> 27
   <211> 20
   <212> ADN
   <213> Homo sapiens
<400> 27
   ttctatggca ggccccttta 20
<210> 28
   <211> 1652
   <212> ADN
   <213> Rattus rattus
<220>
   <221> CDS
   <222> (47) .. (1195)
<400> 28
   cgactctgcc agcatctggc tttggggggc gtgcccgccg cgtaga atg gac agc     55
                                                      Met Asp Ser
                                                       1
   agg gcg cgg agc tct tcc aga aag acc cac ggg aga ggt agc agg tcc   103
   Arg Ala Arg Ser Ser Ser Arg Lys Thr His Gly Arg Gly Ser Arg Ser
       5                   10                  15
   tct tct agg gat gac aag aag tca aag gcc ggg agg ggc aac aga ggc   151
   Ser Ser Arg Asp Asp Lys Lys Ser Lys Ala Gly Arg Gly Asn Arg Gly
   20                  25                  30                  35
   cgc gcg cgc ccg gat gct ggg gca gag cgg cag agc gcc ggg cgg acg   199
   Arg Ala Arg Pro Asp Ala Gly Ala Glu Arg Gln Ser Ala Gly Arg Thr
                   40                  45                  50
   ggg acc cgg gag gag cac cga gct cca gca gcc acg gta gtg aat gtg   247
   Gly Thr Arg Glu Glu His Arg Ala Pro Ala Ala Thr Val Val Asn Val
               55                  60                  65
   gac gag gtt cga agc ccg ggt gag gag ggt acg gaa gtg gtg gcc ctg   295
   Asp Glu Val Arg Ser Pro Gly Glu Glu Gly Thr Glu Val Val Ala Leu
           70                  75                  80
   ctg gag agc gag cga cca gag gaa ggg atc aag ccc tct gga tta ggg   343
   Leu Glu Ser Glu Arg Pro Glu Glu Gly Ile Lys Pro Ser Gly Leu Gly
       85                  90                  95
   gcc tgc gag tgg ctt ctt gtc ctc tcc tcc ctg atc ttc atc atc gta     391
   Ala Cys Glu Trp Leu Leu Val Leu Ser Ser Leu Ile Phe Ile Ile Val
   100                 105                 110                 115
   acg ttt ccc ttt tcc atc tgg ttc tgc ata aag gtt gtt caa gaa tac     439
   Thr Phe Pro Phe Ser Ile Trp Phe Cys Ile Lys Val Val Gln Glu Tyr
                   120                 125                 130
   gaa aga gta att ata ttc cga ctg gga cat ctg ctt cct gga aga gcc     487
   Glu Arg Val Ile Ile Phe Arg Leu Gly His Leu Leu Pro Gly Arg Ala
               135                 140                 145
   aaa gga cct ggc ctg ttc ttt ttt cta ccc tgc ctg gac acc tat cac     535
   Lys Gly Pro Gly Leu Phe Phe Phe Leu Pro Cys Leu Asp Thr Tyr His
           150                 155                 160
   aag gtt gac ctc cgt ctc cag acc ttg gaa ata cct ttc cat gag gtg     583
   Lys Val Asp Leu Arg Leu Gln Thr Leu Glu Ile Pro Phe His Glu Val
       165                 170                 175
   gta acc aaa gat atg ttc aca atg gag ata gac gct gtc tgc tac tac     631
   Val Thr Lys Asp Met Phe Thr Met Glu Ile Asp Ala Val Cys Tyr Tyr
   180                 185                 190                 195
   cgc atg gaa aat gcc tcc ctt ctt cta agc agt cta gct cat gtg tcc     679
   Arg Met Glu Asn Ala Ser Leu Leu Leu Ser Ser Leu Ala His Val Ser
                   200                 205                 210
   aaa gcc atc cag ttc ctg gtg caa acc acc atg aag cgc ctc ttg gca     727
   Lys Ala Ile Gln Phe Leu Val Gln Thr Thr Met Lys Arg Leu Leu Ala
               215                 220                 225
   cat cga tcc ctc act gaa att ctc ctg gaa agg aag agc att gcc caa     775
   His Arg Ser Leu Thr Glu Ile Leu Leu Glu Arg Lys Ser Ile Ala Gln
           230                 235                 240
   gat gta aag gtt gcc ttg gac tca gtg acc tgt gtt tgg ggc atc aaa     823
   Asp Val Lys Val Ala Leu Asp Ser Val Thr Cys Val Trp Gly Ile Lys
       245                 250                 255
   gtg gag aga act gaa att aag gat gta cgg ctg cca gct ggg ctt cag     871
   Val Glu Arg Thr Glu Ile Lys Asp Val Arg Leu Pro Ala Gly Leu Gln
   260                 265                 270                 275
   cac tct ctg gct gtg gaa gct gag gca caa aga cag gcc aaa gtg cgg     919
   His Ser Leu Ala Val Glu Ala Glu Ala Gln Arg Gln Ala Lys Val Arg
                   280                 285                 290
   gtg att gct gcc gaa ggg gaa aaa gct gcc tct gag tcc ctg agg atg     967
   Val Ile Ala Ala Glu Gly Glu Lys Ala Ala Ser Glu Ser Leu Arg Met
               295                 300                 305
   gcg gct gag att ctg tca ggc acc cca gct gct gtc cag ctc cgg tac     1015
   Ala Ala Glu Ile Leu Ser Gly Thr Pro Ala Ala Val Gln Leu Arg Tyr
           310                 315                 320
   ctg cac act ctt cag tcc ttg tcc aca gac aag ccg tcc acc gtg gtt     1063
   Leu His Thr Leu Gln Ser Leu Ser Thr Asp  Lys Pro Ser Thr Val Val
       325                 330                 335
   ttg cct tta ccc ttt gac atg ctg aac ctt ctc tcc tct ccc agc aac   1111
   Leu Pro Leu Pro Phe Asp Met Leu Asn Leu Leu Ser Ser Pro Ser Asn
   340                 345                 350                 355
   aga gca caa gga agc atc aac tac cca agt tct ccc aaa cct gtt gaa   1159
   Arg Ala Gln Gly Ser Ile Asn Tyr Pro Ser Ser Pro Lys Pro Val Glu
                   360                 365                 370
   cca cta aat ccc aaa agg aag gac tct cct atg cta taggggcgag        1205
   Pro Leu Asn Pro Lys Arg Lys Asp Ser Pro Met Leu
               375                 380
   tggacaagag taatgggaat acaccatata aagccgtatc cctgagcgag gcattcggtc 1265
   cccacgccca ggcccaccct gcccttgttg tttgcctttt gagtgtatca tgtcacaaga 1325
   tggacacacg catgagaaca cagtgaaatg gcagagaaga catccagcca cacaagtggg 1385
   tcgtctcatc attcattaca ggaaagaaag agatttagaa ttttgggttg aggggctgga 1445
   gagatggctc agtggttatg aacactgact gctcttccag aggtcctgag ttcaaatccc 1505
   agcaaccaca tggtggctca caaccatctg taatgggatc cgatgccctc ttctggtgtg 1565
   taagacagtg acagtgtact catcatatat aagatgaata aataaaccct tttaaaaaaa 1625
   aaaaaaaaaa aaaaaaaaaa aaaaaaa                                     1652
<210> 29
   <211> 383
   <212> PRT
   <213> Rattus rattus
<400> 29
   Met Asp Ser Arg Ala Arg Ser Ser Ser Arg Lys Thr His Gly Arg Gly
    1               5                  10                  15
   Ser Arg Ser Ser Ser Arg Asp Asp Lys Lys Ser Lys Ala Gly Arg Gly
               20                  25                  30
   Asn Arg Gly Arg Ala Arg Pro Asp Ala Gly Ala Glu Arg Gln Ser Ala
           35                  40                  45
   Gly Arg Thr Gly Thr Arg Glu Glu His Arg Ala Pro Ala Ala Thr Val
       50                  55                  60
   Val Asn Val Asp Glu Val Arg Ser Pro Gly Glu Glu Gly Thr Glu Val
   65                  70                  75                  80
   Val Ala Leu Leu Glu Ser Glu Arg Pro Glu Glu Gly Ile Lys Pro Ser
                   85                  90                  95
   Gly Leu Gly Ala Cys Glu Trp Leu Leu Val Leu Ser Ser Leu Ile Phe
               100                 105                 110
   Ile Ile Val Thr Phe Pro Phe Ser Ile Trp Phe Cys Ile Lys Val Val
           115                 120         125
   Gln Glu Tyr Glu Arg Val Ile Ile Phe Arg Leu Gly His Leu Leu Pro
       130                 135                 140
   Gly Arg Ala Lys Gly Pro Gly Leu Phe Phe Phe Leu Pro Cys Leu Asp
   145                 150                 155                 160
   Thr Tyr His Lys Val Asp Leu Arg Leu Gln Thr Leu Glu Ile Pro Phe
                   165                 170                 175
   His Glu Val Val Thr Lys Asp Met Phe Thr Met Glu Ile Asp Ala Val
               180                 185                 190
   Cys Tyr Tyr Arg Met Glu Asn Ala Ser Leu Leu Leu Ser Ser Leu Ala
           195                 200                 205
   His Val Ser Lys Ala Ile Gln Phe Leu Val Gln Thr Thr Met Lys Arg
       210                 215                 220
   Leu Leu Ala His Arg Ser Leu Thr Glu Ile Leu Leu Glu Arg Lys Ser
   225                 230                 235                 240
   Ile Ala Gln Asp Val Lys Val Ala Leu Asp Ser Val Thr Cys Val Trp
                   245                 250                 255
   Gly Ile Lys Val Glu Arg Thr Glu Ile Lys Asp Val Arg Leu Pro Ala
               260                 265                 270
   Gly Leu Gln His Ser Leu Ala Val Glu Ala Glu Ala Gln Arg Gln Ala
           275                 280                 285
   Lys Val Arg Val Ile Ala Ala Glu Gly Glu Lys Ala Ala Ser Glu Ser
       290                 295                 300
   Leu Arg Met Ala Ala Glu Ile Leu Ser Gly Thr Pro Ala Ala Val Gln
   305                 310                 315                 320
   Leu Arg Tyr Leu His Thr Leu Gln Ser Leu Ser Thr Asp Lys Pro Ser
                   325                 330                 335
   Thr Val Val Leu Pro Leu Pro Phe Asp Met Leu Asn Leu Leu Ser Ser
               340                 345                 350
   Pro Ser Asn Arg Ala Gln Gly Ser Ile Asn Tyr Pro Ser Ser Pro Lys
           355                 360                 365
   Pro Val Glu Pro Leu Asn Pro Lys Arg Lys Asp Ser Pro Met Leu
       370                 375                 380